# EUROPEAN PATENT APPLICATION

(11) **EP 4 840 512 A2**
(43) Date of publication of application: **18.11.2026**
(21) Application number: 26194939.0
(22) Date of filing: 17.12.2020
(51) Int. Cl.: A61P 31/06

(54) **A PHARMACEUTICAL ORAL DOSAGE FORM OF Q203**

(30) Priority: 20.12.2019 US 201962951393 P
(62) Document number(s) of the earlier application(s) in accordance with Art. 76 EPC: 24176555.1 / 4 431 114; 20839277.9 / 4 076 453
(71) Applicant: Qurient Co. Ltd., Seongnam-si, Gyeonggi-do 13487 (KR)
(72) Inventor: NAM, Kiyean, 13554 Seongnam-si (KR); KIM, Jaeseung, 06095 Seoul (KR); JUNG, Chunwon, 16836 Yongin-si (KR); LEE, Saeyeon, 13599 Seongnam-si (KR)
(74) Representative: Vossius & Partner Patentanwälte Rechtsanwälte mbB

(57) **Abstract**

The present invention relates to new pharmaceutical dosage forms of 6-chloro-2-ethyl-N-(4-(4-(4-(trifluoromethoxy)phenyl)piperidine-1-yl)benzyl)imidazo[1,2-a]pyridine-3-carboxamide, in particular of its ditosylate form.

## Description

The present invention relates to new pharmaceutical dosage forms of 6-chloro-2-ethyl-N-(4-(4-(4-(trifluoromethoxy)phenyl)piperidine-1-yl)benzyl)imidazo[1,2-a]pyridine-3-carboxamide, in particular of its ditosylate form.

Tuberculosis as a disease continues to result in millions of deaths each year. Inadequate use of chemotherapy has led to an increasing number of drug resistant cases. This situation is likely to worsen with the emergence of extremely resistant strains to all currently known drugs. Current chemotherapy consists of compounds that directly target Mycobacterium tuberculosis, either by neutralizing general information pathways and critical processes such as RNA polymerization and protein synthesis inhibition or by interfering with mycobacterial specific cell envelop synthesis. The most widely used dedicated anti-tubercular drugs isoniazid, ethionamide, and pyriazin amide are pro-drugs that first require activation. They are administered to a patient for a course of several months. Patients infected with multidrug resistant strains of M. tuberculosis may have to undergo combination therapies for extended periods of time.

WO 2011/113606 describes various anti-tubercular compounds and their use in the treatment of bacterial infections, including compound "Q203" which chemically is 6-chloro-2-ethyl-N-(4-(4-(4-(trifluoromethoxy)phenyl)piperidine-1-yl)benzyl)imidazo[1,2-a]pyridine-3-carboxamide. In a publication by Pethe et al. (Nature Medicine, 19, 1157-1160 (2013), this compound is reported to be active against tuberculosis by interfering with the bacterial energy metabolism, inhibiting cytochrome bc1 activity which is an essential component of the electron transport chain required for synthesis of ATP.

Whilst the compound shows promise for future therapy of tuberculosis and related infections, its administration has been hampered by its rather low solubility in aqueous solutions. This has also made it difficult to manufacture adequate pharmaceutical dosage forms of this compound. Various attempts to formulate this into an appropriate oral dosage form have not been successful.

Accordingly, there is a need in the art to devise and provide a pharmaceutical dosage form that is suitable for oral consumption and that allows the drug to be administered by ingestion.

The present invention addresses these and related needs.

In a first aspect, the present invention relates to a pharmaceutical oral dosage form of 6-chloro-2-ethyl-N-(4-(4-(4-(trifluoromethoxy)phenyl)piperidine-1-yl)benzyl)imidazo[1,2-a]pyridine-3-carboxamide ditosylate, said dosage form comprising a mixture of:
a) granules comprising 6-chloro-2-ethyl-N-(4-(4-(4-(trifluoromethoxy)phenyl)piperidine-1-yl)benzyl)imidazo[1,2-a]pyridine-3-carboxamide ditosylate, at least one intragranular pharmaceutically acceptable excipient, and a solubilizer;
b) a blend surrounding said granules and comprising at least one binder, at least one lubricant and at least one disintegrant, and optionally a filler.

In one embodiment, said at least one intragranular pharmaceutically acceptable excipient is selected from a binder, a filler and a combination of the two.

In one embodiment, said granules (a) consist of 6-chloro-2-ethyl-N-(4-(4-(4-(trifluoromethoxy)phenyl)piperidine-1-yl)benzyl)imidazo[1,2-a]pyridine-3-carboxamide ditosylate, a binder, a filler, and a solubilizer.

In one embodiment, said granules a) do not contain a lubricant and not a disintegrant or superdisintegrant.

In one embodiment, said binder in said granules a) is not: crosslinked starch, microcrystalline cellulose, crosslinked cellulose,
crosslinked polyvinylpyrrolidone (crospovidone), crosslinked alginic acid, soy polysaccharide or calcium silicate.

In one embodiment, said binder in said granules a) is selected from acacia gum, gum tragacanth, gelatin, sucrose, starch, and non-crosslinked polyvinyl pyrrolidone (PVP), preferably non-crosslinked polyvinyl pyrrolidone (PVP).

In one embodiment, said binder in said granules a) is present in said pharmaceutical dosage form in an amount of from about 0.1% by weight to 5% by weight, preferably from about 0.2% by weight to 3.5% by weight, more preferably from about 0.3% by weight to 3% by weight.

In one embodiment, said filler in said granules a) is selected from mannitol, dextrose, dextrin, lactose, sorbitol, sucrose, inositol, and is preferably mannitol.

In one embodiment, said filler in said granules a) is present in said pharmaceutical dosage form in an amount of from about 1.5% by weight to 25% by weight, preferably from about 1.75% by weight to 22.5% by weight, more preferably from about 1.9% by weight to 20% by weight.

In one embodiment, said solubilizer in said granules a) is selected from D-α-tocopherol polyethylene glycol succinate (vitamin E-TPGS), sodium lauryl sulphate (SLS), polyoxy ethylene sorbitan monooleate (Tween^{®}), cetyl triamethyl ammonium bromide (CTAB), amino acids such as glycine, polyethylene glycol, and is preferably D-α-tocopherol polyethylene glycol succinate (vitamin E-TPGS).

In one embodiment, said solubilizer in said granules a) is present in said pharmaceutical dosage form in an amount of from about 0.5% by weight to 8% by weight, preferably from about 0.6% by weight to 7.5% by weight, more preferably from about 0.68% by weight to 6.8% by weight.

The pharmaceutical oral dosage form according to any of the foregoing claims, wherein said granules comprise, preferably consist of, 6-chloro-2-ethyl-N-(4-(4-(4-(trifluoromethoxy) phenyl)piperidine-1-yl)benzyl)imidazo[1,2-a]pyridine-3-carboxamide ditosylate, a binder which is non-crosslinked polyvinyl pyrrolidone (PVP), a filler which is mannitol, and a solubilizer which is D-α-tocopherol polyethylene glycol succinate (vitamin E-TPGS).

In one embodiment, said at least one binder in said blend surrounding said granules (b) is selected from starch, microcrystalline cellulose, povidone, hydroxyl propyl cellulose, hydroxyl propyl methyl cellulose and combinations thereof, and is preferably a binder having also a disintegrant quality, and more preferably is microcrystalline cellulose.

In one embodiment, said at least one binder in said blend surrounding said granules (b) is present in said pharmaceutical dosage form in an amount of from about 25% by weight to 50% by weight, preferably from about 28% by weight to 45% by weight, more preferably from
about 30% by weight to 43% by weight.

In one embodiment, said at least one lubricant in said blend surrounding said granules (b) is selected from magnesium stearate, calcium stearate, PEG6000, sodium stearyl stearate, sodium lauryl sulphate, colloidal silicon dioxide and combinations thereof, wherein, preferably said at least one lubricant in said blend surrounding said granules (b) is a combination of colloidal silicon dioxide and magnesium stearate.

In one embodiment, said at least one lubricant in said blend surrounding said granules (b) is present in said pharmaceutical dosage form in an amount of from about 1% by weight to 5% by weight, preferably from about 0.5% by weight to 4% by weight, more preferably from about 0.75% by weight to 3.5% by weight, wherein, when said at least one lubricant in said blend surrounding said granules (b) is a combination of colloidal silicon dioxide and magnesium stearate, said colloidal silicon dioxide is present in said pharmaceutical dosage form in an amount of from about 0.5% by weight to 3.5% by weight, preferably from about 1% by weight to 2.5% by weight, more preferably about 2% by weight, and wherein in such combination said magnesium stearate is present in said pharmaceutical dosage form in an amount of from about 0.1% by weight to 2% by weight, preferably from about 0.5% by weight to 1.5% by weight, more preferably about 0.8% by weight.

In one embodiment, said at least one disintegrant in said blend surrounding said granules (b) is selected from crosslinked starch, starch glycolate, e.g. sodium starch glycolate, microcrystalline cellulose, crosslinked cellulose, crosslinked polyvinyl pyrrolidone (crospovidone), crosslinked alginic acid, croscarmellose, e.g. croscarmelose sodium or croscarmelose calcium, soy polysaccharide, and calcium silicate.

In one embodiment, said at least one disintegrant in said blend surrounding said granules (b) is present in said pharmaceutical dosage form in an amount of from about 5% by weight to 12% by weight, preferably from about 7.5% by weight to 10% by weight, more preferably from
about 8% by weight to 8.5% by weight.

In one embodiment, said blend surrounding said granules comprises a filler, which preferably is the same filler as the filler comprised in said granules.

In one embodiment, said filler in said blend surrounding said granules is present in said pharmaceutical dosage form in an amount of from about 25% by weight to 50% by weight, preferably from about 27.5% by weight to 45% by weight, more preferably from about 31% by weight to 43% by weight.

In one embodiment, said 6-chloro-2-ethyl-N-(4-(4-(4-(trifluoromethoxy)phenyl)piperidine-1-yl)benzyl)imidazo[1,2-a]pyridine-3-carboxamide ditosylate is present in said pharmaceutical dosage form in an amount of from about 1% by weight to 35% by weight, preferably from
about 2% by weight to 30% by weight, more preferably from about 2.7% by weight to 27.5% by weight.

In one embodiment, the pharmaceutical oral dosage form comprises a mixture of:
a) granules comprising 6-chloro-2-ethyl-N-(4-(4-(4-(trifluoromethoxy)phenyl)piperidine-1-yl)benzyl)imidazo[1,2-a]pyridine-3-carboxamide ditosylate in an amount of about 23 - 30% by weight of the dosage form, mannitol in an amount of about 15 - 25% by weight of the dosage form, polyvinylpyrrolidone in an amount of about 1 - 5% by weight of the dosage form, D-α-tocopherol polyethylene glycol succinate (vitamin E-TPGS) in an amount of about 5 -8% by weight of the dosage form; and
b) blend surrounding said granules surrounding said granules and comprising microcrystalline cellulose in an amount of about 30 - 34% by weight of the dosage form, colloidal silicon dioxide in an amount of about 1 - 3% by weight of the dosage form, magnesium stearate in an amount of about 0.5 - 1.5% by weight of the dosage form, and croscarmellose sodium in an amount of about 7 - 10% by weight of the dosage form;
   or
c) granules comprising 6-chloro-2-ethyl-N-(4-(4-(4-(trifluoromethoxy)phenyl)piperidine-1-yl)benzyl)imidazo[1,2-a]pyridine-3-carboxamide ditosylate in an amount of about 12 - 15% by weight of the dosage form, mannitol in an amount of about 8 - 12% by weight of the dosage form, polyvinylpyrrolidone in an amount of 1 - 2% by weight of the dosage form, D-α-tocopherol polyethylene glycol succinate (vitamin E-TPGS) in an amount of about 2 - 5% by weight of the dosage form; and
d) blend surrounding said granules surrounding said granules and comprising microcrystalline cellulose in an amount of about 30 - 33% by weight of the dosage form, mannitol in an amount of about 30 - 33% by weight of the dosage form, magnesium stearate in an amount of about 0.5 - 1.5% by weight of the dosage form, and croscarmellose sodium in an amount of about 7 - 10% by weight of the dosage form;
   or
e) granules comprising 6-chloro-2-ethyl-N-(4-(4-(4-(trifluoromethoxy)phenyl)piperidine-1-yl)benzyl)imidazo[1,2-a]pyridine-3-carboxamide ditosylate in an amount of about 1 - 5% by weight of the dosage form, mannitol in an amount of about 1 - 3% by weight of the dosage form, polyvinylpyrrolidone in an amount of about 0.1 - 0.5% by weight of the dosage form, D-α-tocopherol polyethylene glycol succinate (vitamin E-TPGS) in an amount of about 0.5 - 0.8% by weight of the dosage form; and
f) blend surrounding said granules surrounding said granules and comprising microcrystalline cellulose in an amount of about 40 - 45% by weight of the dosage form, mannitol in an amount of about 40 - 45% by weight of the dosage form, magnesium stearate in an amount of about 0.5 - 1.5% by weight of the dosage form, and croscarmellose sodium in an amount of about 7 - 10% by weight of the dosage form.

In a further aspect, the present invention relates to a process for preparing a pharmaceutical oral dosage form as defined above, wherein said process comprises:
i. Granulating 6-chloro-2-ethyl-N-(4-(4-(4-(trifluoromethoxy)phenyl)piperidine-1-yl)benzyl)imidazo[1,2-a]pyridine-3-carboxamide ditosylate and the components of said granules a), as defined above, by a suitable granulation process; said suitable granulation process preferably being selected from fluid bed granulation, high shear granulation, fluid bed drying, fluidized spray drying, single pot granulation, steam granulation, thermal adhesion granulation, pneumatic dry granulation, moisture activated dry granulation, melt granulation, freeze granulation, and foam granulation;
ii. Blending the granules obtained in step i. with the components of said blend, as defined above;
iii. Compressing the mixture obtained in step ii. into a pharmaceutical dosage form, preferably a tablet; and, optionally
iv. Coating the dosage form obtained in step iii.

In yet a further aspect, the present invention relates to a pharmaceutical oral dosage form obtainable by a process as defined above.

In still another aspect the present invention relates to a pharmaceutical oral dosage form according to the present invention for use in the treatment of a bacterial infection, preferably tuberculosis or Buruli ulcer.

In yet a further aspect, the present invention also relates to a method of treatment of a bacterial infection, preferably tuberculosis or Buruli ulcer, wherein said pharmaceutical dosage form is administered to a patient in need thereof.

The present inventors have surprisingly found that it is possible to formulate 6-chloro-2-ethyl-N-(4-(4-(4-(trifluoromethoxy)phenyl)piperidine-1-yl)benzyl)imidazo[1,2-a]pyridine-3-carboxamide ditosylate (Q203) into a pharmaceutical oral dosage form. This dosage form can be produced by a robust manufacturing process which provides high uniformity of dosage. Moreover, the pharmaceutical dosage form in accordance with embodiments of the invention can be designed to achieve a highly reproducible disintegration and/or dissolution in aqueous solutions.

The invention is now further described by reference to the following figure which is given to illustrate, not to limit the present invention.

### More specifically,

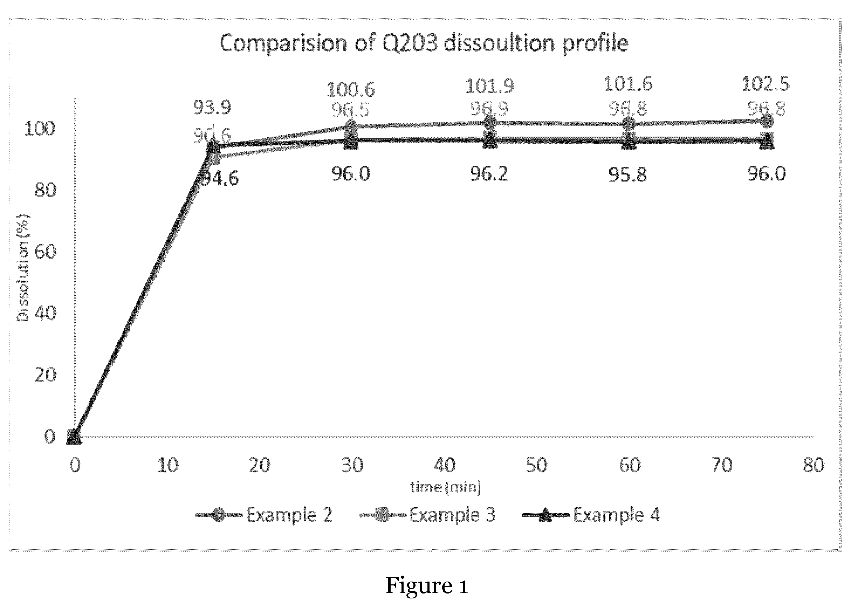

Figure 1 shows a comparison of Q203 dissolution profiles of various tablet formulations shown hereafter.

The invention is now further described by reference to the following examples which are given to illustrate, not to limit the present invention.

### Examples

An exemplary formulation containing Q203 as active ingredient was manufactured. In order to confirm the manufacturability of samples for veriour strengths, low, mid and high concentration of Q203 tablets were produced and were prepared as following genernal procedure: granulation, blending, compression and coating.

### Example 1

### Composition of formulation

| **Ingredients** | **% Level** |
|---|---|
| Q203 | 28.1 |
| Mannitol | 20.7 |
| Microcrystalline Cellulose | 25.7 |
| Croscarmellose Sodium | 3.4 |
| PVP K29/32 | 4.3 |
| Colloidal Silicon Dioxide | 1.4 |
| Vitamin E TPGS | 7.1 |
| Croscarmellose Sodium | 8.4 |
| Magnesium Stearate | 0.9 |
| Total: | 100 |

### Process of Formulation Preparation

1. Screen Q203, Minntol, Microcrystalline Cellulose, Croscarmellose Sodium, Colloidal Silicon Dioxide and PVP K29/32.
2. Add #1 powder into granulator and mix.
3. Add vitamin E-TPGS in water.
4. Mix and dry.
5. Discharge and screen granules
6. Load the blender with the granules, Colloidal Silicon Dioxide, Croscarmellose Sodium, and Magnesium Stearate.
7. Blend for more than 15 minutes.
8. Discharge and compress tablets.

### Example 2

### Composition of formulation

| **Ingredients** | **% Level** |
|---|---|
| Q203 | 28.1 |
| Mannitol | 20.7 |
| Microcrystalline Cellulose | 12.8 |
| Croscarmellose Sodium | 3.4 |
| PVP K29/32 | 4.3 |
| Colloidal Silicon Dioxide | 1.4 |
| Vitamin E TPGS | 7.1 |
| Microcrystalline Cellulose | 12.9 |
| Croscarmellose Sodium | 8.4 |
| Magnesium Stearate | 0.9 |
| Total: | 100 |

### Process of Formulation Preparation

1. Screen Q203, Minntol, Microcrystalline Cellulose, Croscarmellose Sodium, Colloidal Silicon Dioxide and PVP K29/32.
2. Add #1 powder into granulator and mix.
3. Add vitamin E-TPGS in water.
4. Mix and dry.
5. Discharge and screen granules
6. Load the blender with the granules, Microcrystalline Cellulose, Colloidal Silicon Dioxide, Croscarmellose Sodium, and Magnesium Stearate.
7. Blend for more than 15 minutes.
8. Discharge and compress tablets.

### Example 3

### Composition of formulation

| **Ingredients** | **% Level** |
|---|---|
| Q203 | 13.6% |
| Mannitol | 10.0% |
| PVP K29/32 | 1.5% |
| Vitamin E TPGS | 3.4 |
| Microcrystalline Cellulose | 31.3 |
| Mannitol | 31.3 |
| Croscarmellose Sodium | 8.2 |
| Magnesium Stearate | 0.8 |
| Total: | 100 |

### Process of Formulation Preparation

1. Screen Q203, Minntol and PVP K29/32.
2. Add #1 powder into granulator and mix.
3. Add vitamin E-TPGS in water.
4. Mix and dry.
5. Discharge and screen granules
6. Load the blender with the granules, Microcrystalline Cellulose, Mannitol, Croscarmellose Sodium and Magnesium stearate.
7. Blend for more than 15 minutes.
8. Discharge and compress tablets.

### Example 4

### Composition of formulation

| **Ingredients** | **% Level** |
|---|---|
| Q203 | 2.8 |
| Mannitol | 2.1 |
| Microcrystalline Cellulose | 1.3 |
| Croscarmellose Sodium | 0.3 |
| PVP K29/32 | 0.4 |
| Colloidal Silicon Dioxide | 0.1 |
| Vitamin E TPGS | 0.7 |
| Mannitol | 41.3 |
| Microcrystalline Cellulose | 41.3 |
| Croscarmellose Sodium | 8.4 |
| Magnesium Stearate | 1.2 |
| Total: | 100 |

### Process of Formulation Preparation

1. Screen Q203, Minntol, Microcrystalline Cellulose, Croscarmellose Sodium, Colloidal Silicon Dioxide and PVP K29/32.
2. Add #1 powder into granulator and mix.
3. Add vitamin E-TPGS in water.
4. Mix and dry.
5. Discharge and screen granules
6. Load the blender with the granules, Mannitol, Microcrystalline Cellulose, Croscarmellose Sodium and Magnesium Stearate.
7. Blend for more than 15 minutes.
8. Discharge and compress tablets.

### Example 5

### Comparison of Q203 dissolution profiles

A drug-release test which utilizes the USP Apparatus 2 at 75 rpm with 900 mL of 0.1N HCl, was used to study the in vitro release at 37 °C ± 0.5 °C. In the guidance about dissolution testing of immediate release solid oral dosage forms, for BCS class 2 products which are slowly dissolving or poorly water soluble drugs, it was recommended to characterize the quality of the product to ensure 85% dissolution. Manufactured Q203 tablets in accordance with examples 2, 3 and 4 showed to dissolve quickly; and complete dissolution (> 90%) at 15 min is observed for all three batches. So those formulation are appropriate formulations and achieve a highly reproducible disintegration and/or dissolution.. Results are shown in the following table and in Figure 1. (It should be noted that a dissolution > 100% means that such dissolution is higher than the dissolution of the respective control formulation used in the dissolution test.)

| Time (min) | Example 2 [ %dissolution] | Example 3 [% dissolution] | Example 4 [% dissolution] |
|---|---|---|---|
| 0 | 0 | 0 | 0 |
| 15 | 93.9 | 90.6 | 94.6 |
| 30 | 100.6 | 96.5 | 96 |
| 45 | 101.9 | 96.9 | 96.2 |
| 60 | 101.6 | 96.8 | 95.8 |
| 75 | 102.5 | 96.8 | 96 |

The present invention particularly relates to the following items:
1. A pharmaceutical oral dosage form of 6-chloro-2-ethyl-N-(4-(4-(4-(trifluoromethoxy)phenyl)piperidine-1-yl)benzyl)imidazo[1,2-a]pyridine-3-carboxamide ditosylate, said dosage form comprising a mixture of:
   a) granules comprising 6-chloro-2-ethyl-N-(4-(4-(4-(trifluoromethoxy)phenyl)piperidine-1-yl)benzyl)imidazo[1,2-a]pyridine-3-carboxamide ditosylate, at least one intragranular pharmaceutically acceptable excipient, and a solubilizer;
   b) a blend surrounding said granules and comprising at least one binder, at least one lubricant and at least one disintegrant, and optionally a filler.
2. The pharmaceutical oral dosage form according to item 1, wherein said at least one intragranular pharmaceutically acceptable excipient is selected from a binder, a filler and a combination of the two.
3. The pharmaceutical oral dosage form according to any of items 1 and 2, wherein said granules (a)) consist of 6-chloro-2-ethyl-*N*-(4-(4-(4-(trifluoromethoxy)phenyl)piperidine-1-yl)benzyl)imidazo[1,2-a]pyridine-3-carboxamide ditosylate, a binder, a filler, and a solubilizer.
4. The pharmaceutical oral dosage form according to any of the foregoing items, wherein said granules a) do not contain a lubricant and not a disintegrant or superdisintegrant.
5. The pharmaceutical oral dosage form according to any of items 2 - 4, wherein said binder in said granules a) is not: crosslinked starch, microcrystalline cellulose, crosslinked cellulose, crosslinked polyvinylpyrrolidone (crospovidone), crosslinked alginic acid, soy polysaccharide or calcium silicate.
6. The pharmaceutical oral dosage form according to any of items 2 - 5, wherein said binder in said granules a) is selected from acacia gum, gum tragacanth, gelatin, sucrose, starch, and non-crosslinked polyvinylpyrrolidone (PVP), preferably non-crosslinked polyvinylpyrrolidone (PVP).
7. The pharmaceutical oral dosage form according to item 6, wherein said binder in said granules a) is present in said pharmaceutical dosage form in an amount of from about 0.1% by weight to 5% by weight, preferably from about 0.2% by weight to 3.5% by weight, more preferably from about 0.3% by weight to 3% by weight.
8. The pharmaceutical oral dosage form according to any of items 2 - 7, wherein said filler in said granules a) is selected from mannitol, dextrose, dextrin, lactose, sorbitol, sucrose, inositol, and is preferably mannitol.
9. The pharmaceutical oral dosage form according to item 8, wherein said filler in said granules a) is present in said pharmaceutical dosage form in an amount of from about 1.5% by weight to 25% by weight, preferably from about 1.75% by weight to 22.5% by weight, more preferably from about 1.9% by weight to 20% by weight.
10. The pharmaceutical oral dosage form according to any of the foregoing items, wherein said solubilizer in said granules a) is selected from D-α-tocopherol polyethylene glycol succinate (vitamin E-TPGS), sodium lauryl sulphate (SLS), polyoxyethylene sorbitan monooleate (Tween^{®}), cetyltriamethylammonium bromide (CTAB), amino acids such as glycine, polyethylene glycol, and is preferably D-α-tocopherol polyethylene glycol succinate (vitamin E-TPGS).
11. The pharmaceutical oral dosage form according to item 10, wherein said solubilizer in said granules a) is present in said pharmaceutical dosage form in an amount of from about 0.5% by weight to 8% by weight, preferably from about 0.6% by weight to 7.5% by weight, more preferably from about 0.68% by weight to 6.8% by weight.
12. The pharmaceutical oral dosage form according to any of the foregoing items, wherein said granules comprise, preferably consist of, 6-chloro-2-ethyl-*N*-(4-(4-(4-(trifluoromethoxy)phenyl)piperidine-1-yl)benzyl)imidazo[1,2-a]pyridine-3-carboxamide ditosylate, a binder which is non-crosslinked polyvinylpyrrolidone (PVP), a filler which is mannitol, and a solubilizer which is D-α-tocopherol polyethylene glycol succinate (vitamin E-TPGS).
13. The pharmaceutical oral dosage form according to any of the foregoing items, wherein said at least one binder in said blend surrounding said granules (b)) is selected from starch, microcrystalline cellulose, povidone, hydroxypropyl cellulose, hydroxypropylmethyl cellulose and combinations thereof, and is preferably a binder having also a disintegrant quality, and more preferably is microcrystalline cellulose.
14. The pharmaceutical oral dosage form according to item 13, wherein said at least one binder in said blend surrounding said granules (b)) is present in said pharmaceutical dosage form in an amount of from about 25% by weight to 50% by weight, preferably from about 28% by weight to 45% by weight, more preferably from about 30% by weight to 43% by weight.
15. The pharmaceutical oral dosage form according to any of the foregoing items, wherein said at least one lubricant in said blend surrounding said granules (b)) is selected from magnesium stearate, calcium stearate, PEG6000, sodium stearyl stearate, sodium lauryl sulphate, colloidal silicon dioxide and combinations thereof, wherein preferably said at least one lubricant in said blend surrounding said granules (b)) is a combination of colloidal silicon dioxide and magnesium stearate.
16. The pharmaceutical oral dosage form according to item 15, wherein said at least one lubricant in said blend surrounding said granules (b)) is present in said pharmaceutical dosage form in an amount of from about 1% by weight to 5% by weight, preferably from about 0.5% by weight to 4% by weight, more preferably from about 0.75% by weight to 3.5% by weight, wherein, when said said at least one lubricant in said blend surrounding said granules (b)) is a combination of colloidal silicon dioxide and magnesium stearate, said colloidal silicon dioxide is present in said pharmaceutical dosage form in an amount of from about 0.5% by weight to 3.5% by weight, preferably from about 1% by weight to 2.5% by weight, more preferably about 2% by weight, and wherein in such combination said magnesium stearate is present in said pharmaceutical dosage form in an amount of from about 0.1% by weight to 2% by weight, preferably from about 0.5% by weight to 1.5% by weight, more preferably about 0.8% by weight.
17. The pharmaceutical oral dosage form according to any of the foregoing items, wherein said at least one disintegrant in said blend surrounding said granules (b)) is selected from crosslinked starch, starch glycolate, e.g. sodium starch glycolate, microcrystalline cellulose, crosslinked cellulose, crosslinked polyvinylpyrrolidone (crospovidone), crosslinked alginic acid, croscarmellose, e.g. croscarmelose sodium or croscarmelose calcium, soy polysaccharide, and calcium silicate.
18. The pharmaceutical oral dosage form according to item 17, wherein said at least one disintegrant in said blend surrounding said granules (b)) is present in said pharmaceutical dosage form in an amount of from about 5% by weight to 12% by weight, preferably from about 7.5% by weight to 10% by weight, more preferably from about 8% by weight to 8.5% by weight.
19. The pharmaceutical oral dosage form according to any of items 2 - 18, wherein said blend surrounding said granules comprises a filler, which preferably is the same filler as the filler comprised in said granules.
20. The pharmaceutical oral dosage form according to item 19, wherein said filler in said blend surrounding said granules is present in said pharmaceutical dosage form in an amount of from about 25% by weight to 50% by weight, preferably from about 27.5% by weight to 45% by weight, more preferably from about 31% by weight to 43% by weight.
21. The pharmaceutical oral dosage form according to any of the foregoing items, wherein said 6-chloro-2-ethyl-*N*-(4-(4-(4-(trifluoromethoxy)phenyl)piperidine-1-yl)benzyl)imidazo[1,2-a]pyridine-3-carboxamide ditosylate is present in said pharmaceutical dosage form in an amount of from about 1% by weight to 35% by weight, preferably from about 2% by weight to 30% by weight, more preferably from about 2.7% by weight to 27.5% by weight.
22. The pharmaceutical oral dosage form according to any of the foregoing items, comprising a mixture of:
   a) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate in an amount of about 23 - 30% by weight of the dosage form, mannitol in an amount of about 15 - 25% by weight of the dosage form, polyvinylpyrrolidone in an amount of about 1 - 5% by weight of the dosage form, D-α-tocopherol polyethylene glycol succinate (vitamin E-TPGS) in an amount of about 5-8% by weight of the dosage form; and
   b) a blend surrounding said granules and comprising microcrystalline cellulose in an amount of about 30 - 34% by weight of the dosage form, colloidal silicon dioxide in an amount of about 1 - 3% by weight of the dosage form, magnesium stearate in an amount of about 0.5 - 1.5% by weight of the dosage form, and croscarmellose sodium in an amount of about 7 - 10% by weight of the dosage form; or
   c) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate in an amount of about 12 - 15% by weight of the dosage form, mannitol in an amount of about 8 - 12% by weight of the dosage form, polyvinylpyrrolidone in an amount of 1 - 2% by weight of the dosage form, D-α-tocopherol polyethylene glycol succinate (vitamin E-TPGS) in an amount of about 2 - 5% by weight of the dosage form; and
   d) a blend surrounding said granules and comprising microcrystalline cellulose in an amount of about 30 - 33% by weight of the dosage form, mannitol in an amount of about 30 - 33% by weight of the dosage form, magnesium stearate in an amount of about 0.5 - 1.5% by weight of the dosage form, and croscarmellose sodium in an amount of about 7 - 10% by weight of the dosage form; or
   e) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate in an amount of about 1 - 5% by weight of the dosage form, mannitol in an amount of about 1 - 3% by weight of the dosage form, polyvinylpyrrolidone in an amount of about 0.1 - 0.5% by weight of the dosage form, D-α-tocopherol polyethylene glycol succinate (vitamin E-TPGS) in an amount of about 0.5 - 0.8% by weight of the dosage form; and
   f) a blend surrounding said granules and comprising microcrystalline cellulose in an amount of about 40 - 45% by weight of the dosage form, mannitol in an amount of about 40 - 45% by weight of the dosage form, magnesium stearate in an amount of about 0.5 - 1.5% by weight of the dosage form, and croscarmellose sodium in an amount of about 7 - 10% by weight of the dosage form.
23. A process for preparing a pharmaceutical oral dosage form as defined in any of items 1- 22, wherein said process comprises:
   i. Granulating 6-chloro-2-ethyl-*N*-(4-(4-(4-(trifluoromethoxy)phenyl)piperidine-1-yl)benzyl)imidazo[1,2-a]pyridine-3-carboxamide ditosylate and the components of said granules a), as defined in any of items 1 - 22, by a suitable granulation process; said suitable granulation process preferably being selected from fluid bed granulation, high shear granulation, fluid bed drying, fluidized spray drying, single pot granulation, steam granulation, thermal adhesion granulation, pneumatic dry granulation, moisture activated dry granulation, melt granulation, freeze granulation, and foam granulation;
   ii. Blending the granules obtained in step i. with the components of said blend, as defined in any of items 1 - 22;
   iii. Compressing the mixture obtained in step ii. into a pharmaceutical dosage form, preferably a tablet; and, optionally
   iv. Coating the dosage form obtained in step iii.
24. A pharmaceutical oral dosage form obtainable by a process as defined in item 23.
25. A pharmaceutical oral dosage form according to any of items 1 - 22 or 24 for use in the treatment of a bacterial infection, preferably tuberculosis or Buruli ulcer.

## Claims

1. A pharmaceutical oral dosage form of 6-chloro-2-ethyl-*N*-(4-(4-(4-(trifluoromethoxy)phenyl)piperidine-1-yl)benzyl)imidazo[1,2-a]pyridine-3-carboxamide ditosylate, said dosage form comprising a mixture of:
a) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate, at least one intragranular pharmaceutically acceptable excipient, and a solubilizer, wherein said at least one intragranular pharmaceutically acceptable excipient comprises a filler;
b) a blend surrounding said granules and comprising at least one binder, at least one lubricant and at least one disintegrant, and optionally a filler.

2. The pharmaceutical oral dosage form according to claim 1, wherein said at least one intragranular pharmaceutically acceptable excipient is a combination of a binder and a filler, and further wherein:
(i) said binder in said granules a) is not: microcrystalline cellulose, crosslinked cellulose, crosslinked polyvinylpyrrolidone (crospovidone), crosslinked alginic acid, soy polysaccharide or calcium silicate; and/or
(ii) said binder in said granules a) is selected from acacia gum, gum tragacanth, gelatin, sucrose, starch, and non-crosslinked polyvinylpyrrolidone (PVP), preferably non-crosslinked polyvinylpyrrolidone (PVP),
wherein it is preferred that said binder in said granules a) is present in said pharmaceutical dosage form in an amount of from about 0.1% by weight to 5% by weight, more preferably from about 0.2% by weight to 3.5% by weight, even more preferably from about 0.3% by weight to 3% by weight.

3. The pharmaceutical oral dosage form according to claim 1 or 2, wherein said filler in said granules a) is selected from mannitol, dextrose, dextrin, lactose, sorbitol, sucrose, inositol, and is preferably mannitol.

4. The pharmaceutical oral dosage form according to claim 3, wherein said filler in said granules a) is present in said pharmaceutical dosage form in an amount of from about 1.5% by weight to 25% by weight, preferably from about 1.75% by weight to 22.5% by weight, more preferably from about 1.9% by weight to 20% by weight.

5. The pharmaceutical oral dosage form according to any of the foregoing claims, wherein said solubilizer in said granules a) is selected from D-α-tocopherol polyethylene glycol succinate (vitamin E-TPGS), sodium lauryl sulphate (SLS), polyoxyethylene sorbitan monooleate (Tween^{®}), cetyltriamethylammonium bromide (CTAB), amino acids such as glycine, polyethylene glycol, and is preferably D-α-tocopherol polyethylene glycol succinate (vitamin E-TPGS);
wherein it is preferred that said solubilizer in said granules a) is present in said pharmaceutical dosage form in an amount of from about 0.5% by weight to 8% by weight, more preferably from about 0.6% by weight to 7.5% by weight, even more preferably from about 0.68% by weight to 6.8% by weight.

6. The pharmaceutical oral dosage form according to any of the foregoing claims, wherein said granules consist of 6-chloro-2-ethyl-*N*-(4-(4-(4-(trifluoromethoxy)phenyl)piperidine-1-yl)benzyl)imidazo[1,2-a]pyridine-3-carboxamide ditosylate, a binder which is non-crosslinked polyvinylpyrrolidone (PVP), a filler which is mannitol, and a solubilizer which is D-α-tocopherol polyethylene glycol succinate (vitamin E-TPGS).

7. The pharmaceutical oral dosage form according to any of the foregoing claims, wherein:
(i) said at least one binder in said blend surrounding said granules b) is selected from starch, microcrystalline cellulose, povidone, hydroxypropyl cellulose, hydroxypropylmethyl cellulose and combinations thereof, and is preferably a binder having also a disintegrant quality, and more preferably is microcrystalline cellulose;
wherein it is preferred that said at least one binder in said blend surrounding said granules b) is present in said pharmaceutical dosage form in an amount of from about 25% by weight to 50% by weight, more preferably from about 28% by weight to 45% by weight, even more preferably from about 30% by weight to 43% by weight; and/or
(ii) said at least one lubricant in said blend surrounding said granules b) is selected from magnesium stearate, calcium stearate, PEG6000, sodium stearyl stearate, sodium lauryl sulphate, colloidal silicon dioxide and combinations thereof, wherein preferably said at least one lubricant in said blend surrounding said granules b) is a combination of colloidal silicon dioxide and magnesium stearate;
wherein it is preferred that said at least one lubricant in said blend surrounding said granules b) is present in said pharmaceutical dosage form in an amount of from about 1% by weight to 5% by weight, more preferably from about 0.5% by weight to 4% by weight, even more preferably from about 0.75% by weight to 3.5% by weight, wherein, when said at least one lubricant in said blend surrounding said granules b) is a combination of colloidal silicon dioxide and magnesium stearate, said colloidal silicon dioxide is present in said pharmaceutical dosage form in an amount of from about 0.5% by weight to 3.5% by weight, preferably from about 1% by weight to 2.5% by weight, more preferably about 2% by weight, and wherein in such combination said magnesium stearate is present in said pharmaceutical dosage form in an amount of from about 0.1% by weight to 2% by weight, preferably from about 0.5% by weight to 1.5% by weight, more preferably about 0.8% by weight; and/or
(iii) said at least one disintegrant in said blend surrounding said granules b) is selected from crosslinked starch, starch glycolate, e.g. sodium starch glycolate, microcrystalline cellulose, crosslinked cellulose, crosslinked polyvinylpyrrolidone (crospovidone), crosslinked alginic acid, croscarmellose, e.g. croscarmelose sodium or croscarmelose calcium, soy polysaccharide, and calcium silicate;
wherein it is preferred that said at least one disintegrant in said blend surrounding said granules b) is present in said pharmaceutical dosage form in an amount of from about 5% by weight to 12% by weight, more preferably from about 7.5% by weight to 10% by weight, even more preferably from about 8% by weight to 8.5% by weight.

8. The pharmaceutical oral dosage form according to any of claims 1 - 7, wherein said blend surrounding said granules comprises a filler, which preferably is the same filler as the filler comprised in said granules.

9. The pharmaceutical oral dosage form according to claim 8, wherein said filler in said blend surrounding said granules is present in said pharmaceutical dosage form in an amount of from about 25% by weight to 50% by weight, preferably from about 27.5% by weight to 45% by weight, more preferably from about 31% by weight to 43% by weight.

10. The pharmaceutical oral dosage form according to any of the foregoing claims, wherein said 6-chloro-2-ethyl-*N*-(4-(4-(4-(trifluoromethoxy)phenyl)piperidine-1-yl)benzyl)imidazo[1,2-a]pyridine-3-carboxamide ditosylate is present in said pharmaceutical dosage form in an amount of from about 1% by weight to 35% by weight, preferably from about 2% by weight to 30% by weight, more preferably from about 2.7% by weight to 27.5% by weight.

11. The pharmaceutical oral dosage form according to any of the foregoing claims, comprising a mixture of:
a) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate in an amount of about 23 - 30% by weight of the dosage form, mannitol in an amount of about 15 - 25% by weight of the dosage form, polyvinylpyrrolidone in an amount of about 1 - 5% by weight of the dosage form, D-α-tocopherol polyethylene glycol succinate (vitamin E-TPGS) in an amount of about 5-8% by weight of the dosage form; and
b) a blend surrounding said granules and comprising microcrystalline cellulose in an amount of about 30 - 34% by weight of the dosage form, colloidal silicon dioxide in an amount of about 1 - 3% by weight of the dosage form, magnesium stearate in an amount of about 0.5 - 1.5% by weight of the dosage form, and croscarmellose sodium in an amount of about 7 - 10% by weight of the dosage form.

12. The pharmaceutical oral dosage form according to any of the foregoing claims, comprising a mixture of:
c) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate in an amount of about 12 - 15% by weight of the dosage form, mannitol in an amount of about 8 - 12% by weight of the dosage form, polyvinylpyrrolidone in an amount of 1 - 2% by weight of the dosage form, D-α-tocopherol polyethylene glycol succinate (vitamin E-TPGS) in an amount of about 2 - 5% by weight of the dosage form; and
d) a blend surrounding said granules and comprising microcrystalline cellulose in an amount of about 30 - 33% by weight of the dosage form, mannitol in an amount of about 30 - 33% by weight of the dosage form, magnesium stearate in an amount of about 0.5 - 1.5% by weight of the dosage form, and croscarmellose sodium in an amount of about 7 - 10% by weight of the dosage form.

13. The pharmaceutical oral dosage form according to any of the foregoing claims, comprising a mixture of:
e) granules comprising 6-chloro-2-ethyl-*N*-(4-(4-(4-(trifluoromethoxy)phenyl)piperidine-1-yl)benzyl)imidazo[1,2-a]pyridine-3-carboxamide ditosylate in an amount of about 1 - 5% by weight of the dosage form, mannitol in an amount of about 1 - 3% by weight of the dosage form, polyvinylpyrrolidone in an amount of about 0.1 - 0.5% by weight of the dosage form, D-α-tocopherol polyethylene glycol succinate (vitamin E-TPGS) in an amount of about 0.5 - 0.8% by weight of the dosage form; and
f) a blend surrounding said granules and comprising microcrystalline cellulose in an amount of about 40 - 45% by weight of the dosage form, mannitol in an amount of about 40 - 45% by weight of the dosage form, magnesium stearate in an amount of about 0.5 - 1.5% by weight of the dosage form, and croscarmellose sodium in an amount of about 7 - 10% by weight of the dosage form.

14. A process for preparing a pharmaceutical oral dosage form as defined in any of claims 1 - 13, wherein said process comprises:
i. granulating 6-chloro-2-ethyl-*N*-(4-(4-(4-(trifluoromethoxy)phenyl)piperidine-1-yl)benzyl)imidazo[1,2-a]pyridine-3-carboxamide ditosylate and the components of said granules a), as defined in any of claims 1 - 13, by a suitable granulation process; said suitable granulation process preferably being selected from fluid bed granulation, high shear granulation, fluid bed drying, fluidized spray drying, single pot granulation, steam granulation, thermal adhesion granulation, pneumatic dry granulation, moisture activated dry granulation, melt granulation, freeze granulation, and foam granulation;
ii. blending the granules obtained in step i. with the components of said blend, as defined in any of claims 1 - 13;
iii. compressing the mixture obtained in step ii. into a pharmaceutical dosage form, preferably a tablet; and, optionally
iv. coating the dosage form obtained in step iii.

15. A pharmaceutical oral dosage form according to any of claims 1 - 13 for use in the treatment of a bacterial infection, preferably tuberculosis or Buruli ulcer.
